# FASCICULE DE BREVET EUROPEEN

(11) **EP 1 643 922 B1**
(45) Date de publication et mention de la délivrance du brevet: **16.03.2011**
(21) Numéro de dépôt: 04767362.9
(22) Date de dépôt: 17.06.2004
(51) Int. Cl.: A61B 17/70

(54) **CROCHETS PEDICULAIRES POUR DISPOSITIF D'ANCRAGE RACHIDIEN**
PEDIKELHAKEN FÜR EINE RACHIDIALE FIXIERVORRICHTUNG
PEDICLE HOOKS FOR A RACHIDIAL FIXING DEVICE

(30) Priorité: 17.06.2003 FR 0307253
(43) Date de publication de la demande: 12.04.2006
(73) Titulaire: Surgiview, 75002 Paris (FR)
(72) Inventeur: STEIB, Jean-Paul, F-67100 Strasbourg (FR); LEROY, Jean-Yves, F-62870 Campagne Les Hesdin (FR); LOURDEL, Rodolphe, F-62660 Beuvry (FR); ROKEGEM, Pascal, F-62000 Arras (FR); VIART, Guy, F-62128 Saint Leger (FR)
(74) Mandataire: Schmitt, John
(86) Numéro de dépôt international: PCT/FR2004/001500
(87) Numéro de publication internationale: WO 2004/112626

(56) Documents cités:
- EP-A- 0 558 121
- FR-A- 2 813 782
- US-A- 5 498 263
- US-A- 6 077 263
- US-A1- 2003 045 874

## Description

La présente invention est relative à une paire de crochets pédiculaires pour dispositif d'ancrage rachidien et plus particulièrement pour le guidage et la retenue des tiges de liaison dudit dispositif au niveau de chaque vertèbre de la colonne vertébrale.

On connait d'après le brevet US 6, 077, 263 (Ce document montre l'état de la technique le plus proche de l'object de la revendication 1) un dispositif d'ancrage rachidien à crochets, l'un des crochets est destiné à recevoir d'une part une tige de liaison et d'autre part suivant une même direction longitudinale que celle de ladite tige de liaison une seconde tige permettant de relier le premier crochet au second crochet.

On note que le dispositif rachidien décrit dans le brevet US 6, 077, 263 ne permet pas du fait de l'agencement des tiges de liaison une mise en compression des deux crochets en direction de la vertèbre correspondante.

On connait d'après le brevet US 6, 077, 263 un dispositif rachidien comportant des connecteurs fixés dans le corps osseux des vertèbres au moyen de vis d'ancrage. Les connecteurs sont reliés entre eux d'une part au moyen de plaque suivant une première direction longitudinale et d'autre part au moyen de tiges transversales suivant une direction perpendiculaire à la première.

On constate que le dispositif rachidien décrit dans le brevet US 6, 077, 263 ne permet pas, du fait de la fixation des connecteurs par des vis d'ancrage dans le corps des vertèbres, une mise en compressions de ces dernières au moyen de la tige transversale.

On connait également d'après le brevet EP 0558121 un dispositif de fixation cervicale constitué de crochets pédiculaires reliés entre eux par un assemblage de tiges parallèles deux à deux. On note que la position des crochets pédiculaires ne permet pas leur mise en compression au moyen de tiges d'assemblage en direction du centre de la vertèbre correspondante.

Le dispositif d'ancrage rachidien suivant la présente invention est fixé sur chaque vertèbre d'un segment rachidien à instrumenter d'une colonne vertébrale après résection des pédicules transverses et de l'épineuse.

Le dispositif d'ancrage rachidien suivant la présente invention est retenu sur chaque vertèbre par une mise en compression des deux crochets pédiculaires en direction du centre de ladite vertèbre. Les crochets pédiculaires viennent prendre appui respectivement sur la base des pédicules pour assurer une bonne tenue du dispositif d'ancrage rachidien.

Le dispositif d'ancrage rachidien à crochets pédiculaires permettant la mise en place et la fixation d'une tige de liaison au niveau de chaque vertèbre d'une colonne vertébrale comprend des crochets pédiculaires pourvus de moyens de fixation pour la réception et le blocage d'une tige transversale reliant lesdits crochets pédiculaires entre eux et permettant une mise en compression des deux crochets pédiculaires en direction du centre de la vertèbre.

Le dispositif d'ancrage rachidien suivant la présente invention comporte un premier crochet pédiculaire comprenant une partie d'ancrage osseux et un corps ouvert pour la réception et la fixation de la tige de liaison au moyen d'un clip par l'intermédiaire d'une vis de pression, ledit corps ouvert comportant sur sa face externe un prolongement pourvu de moyens de fixation pour la réception et le blocage de la tige transversale et un second crochet pédiculaire comprenant une partie d'ancrage osseux et un corps ouvert pourvu de moyens de fixation pour la réception et la fixation de la tige transversale.

La description qui va suivre en regard des dessins annexés, donnés à titre d'exemples non limitatifs, permettra de mieux comprendre l'invention, les caractéristiques qu'elle présente et les avantages qu'elle est susceptible de procurer :
Figure 1 est une vue en perspective éclatée montrant un dispositif d'ancrage rachidien à crochets pédiculaires suivant la présente invention.
Figure 2 est une vue en perspective illustrant le dispositif d'ancrage rachidien à crochets pédiculaires en position assemblée suivant la présente invention.
Figure 3 est une vue en perspective représentant le dispositif d'ancrage rachidien à crochets pédiculaires en position montée sur des corps vertébraux d'une colonne vertébrale.
Figures 4 et 5 sont des vues en perspective montrant une première variante du dispositif d'ancrage rachidien à crochets pédiculaires suivant la présente invention.
Figure 6 est une vue en perspective illustrant une variante d'un écrou de serrage du dispositif d'ancrage rachidien à crochets pédiculaires suivant la présente invention.
Figures 7 et 8 sont des vues en perspective représentant une autre variante du dispositif d'ancrage rachidien à crochets pédiculaires suivant la présente invention.

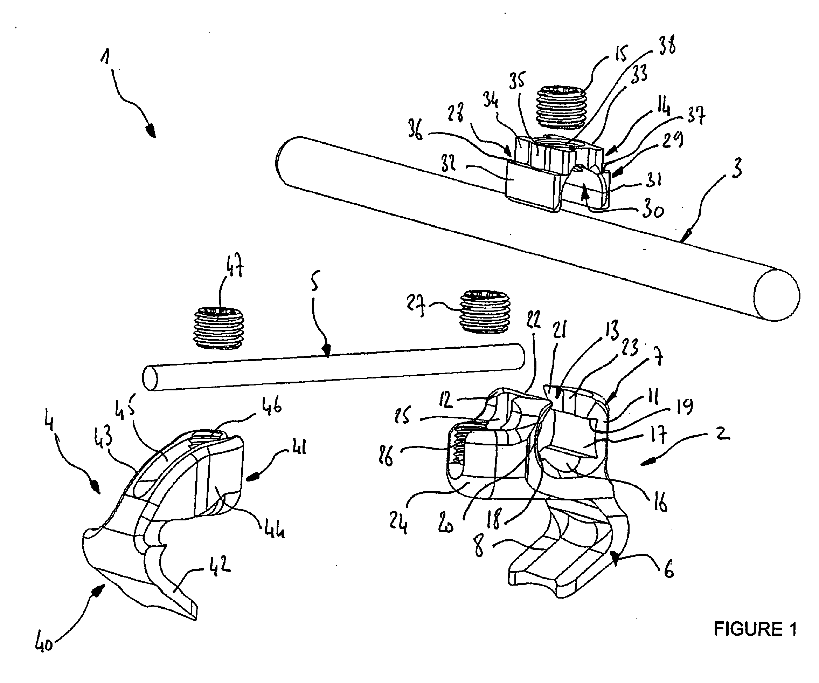

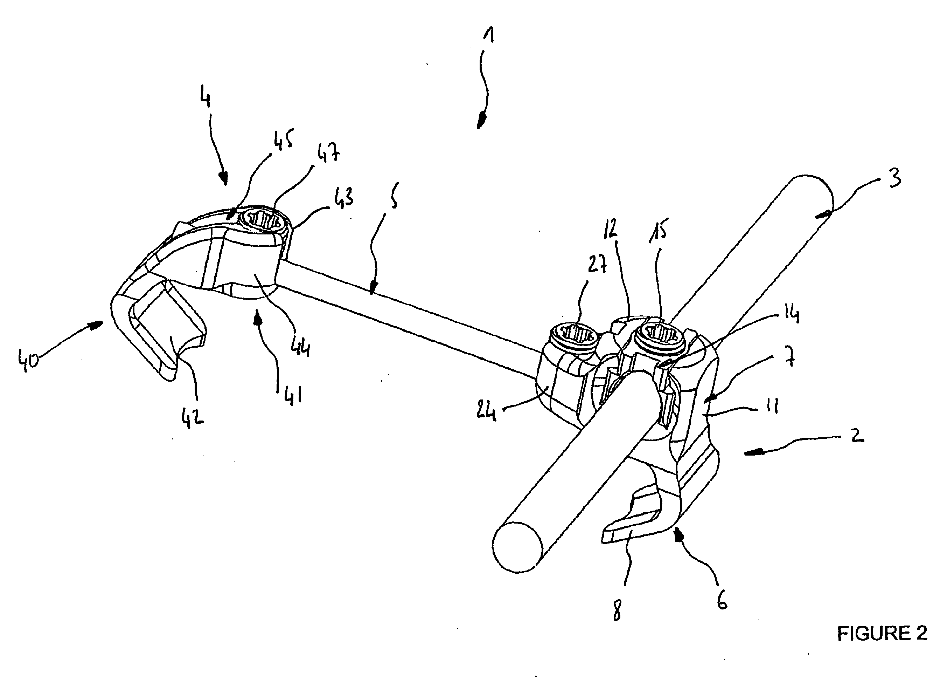

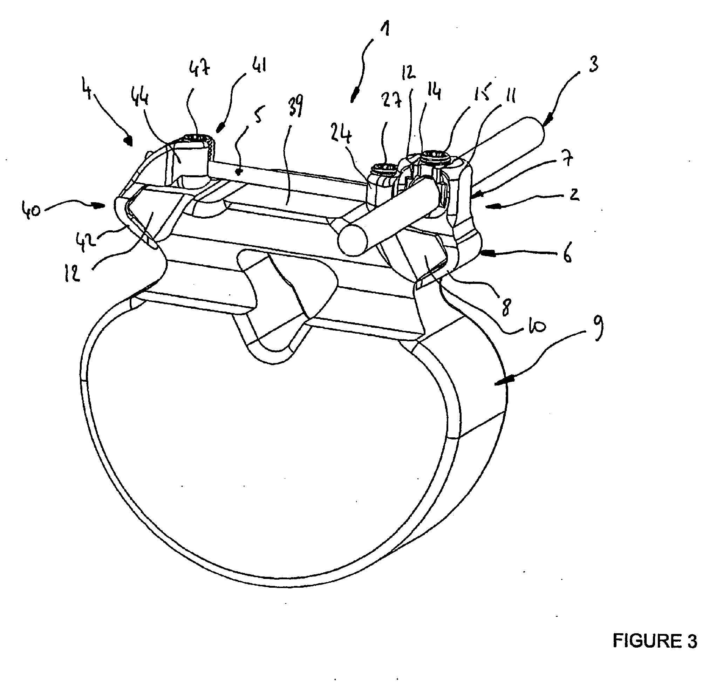

On a montré en figure 1 à 3 un dispositif d'ancrage rachidien 1 comportant un premier crochet pédiculaire 2 destiné à recevoir une tige de liaison 3 et un second crochet pédiculaire 4 relié au premier par une tige transversale 5 présentant un profil cylindrique sécable et lisse.

Le premier crochet pédiculaire 2 comprend une partie d'ancrage osseux 6 se prolongeant par un corps ouvert 7 pour la réception et la fixation de la tige de liaison 3.

La partie d'ancrage osseux 6 est constituée d'une lame 8 en forme de crochet s'étendant en dessous du corps 7 pour venir s'accrocher et se fixer sur une vertèbre 9 d'une colonne vertébrale et plus particulièrement sur un pédicule transverse 10 après résection.

La lame 8 en forme de crochet est disposée en dessous du corps ouvert 7 dans un même plan vertical de manière que la zone d'accrochage sur le pédicule transverse 10 soit sensiblement perpendiculaire à la direction de la tige de liaison 3.

Le corps ouvert 7 du premier crochet pédiculaire 2 comporte deux branches verticales et latérales 11, 12 délimitant une ouverture 13 en forme de U, ménagée pour recevoir un clip 14 muni d'une vis de pression 15 pour le blocage, dans le fond de l'ouverture 13, de la tige de liaison 3.

Les branches verticales 11 et 12 comportent, sur leurs faces internes et au dessus du fond 16 de l'ouverture 13, des rainures opposées 17, 18 délimitant, au moins dans leur partie supérieure, une face d'épaulement plane 19, 20.

Les branches verticales 11 et 12 comportent, dans un plan perpendiculaire au dessus et dans un plan perpendiculaire des faces d'épaulement 19, 20, des faces planes 21, 22 présentant chacune un évidement 23 en forme d'arc de cercle.

La branche verticale 12 du corps ouvert 7 comprend sur sa face externe un prolongement 24 dirigé d'une part perpendiculairement à la direction de fixation de la tige de liaison 3 dans l'ouverture 13, et d'autre part au dessus de la lame 8 formant crochet.

Le prolongement 24 comporte un logement allongé 25 en forme de U débouchant à l'extérieur du prolongement et présentant suivant une direction verticale une partie filetée 26 coopérant avec une vis de pression 27 pour le blocage de la tige transversale 5 dans ledit logement.

Le clip 14, coopérant par coulissement latéral avec l'ouverture 13 du corps ouvert 7, comprend deux parois verticales et latérales 28, 29 délimitant une ouverture 30 en forme de U ménagée pour recevoir la tige de liaison 3.

Les parois verticales 28, 29 comportent, d'une part des premières faces planes 31, 32 destinées à coopérer respectivement avec les rainures 17, 18 et d'autre part, des secondes faces planes 33, 34 présentant chacune une protubérance prismatique 35 coopérant avec l'évidemment 23 des faces 21, 22 des branches verticales 11 et 12.

Les premières faces planes 31, 32 et les secondes faces planes 33, 34 des parois verticales 28, 29 sont respectivement décalées les unes des autres pour constituer des épaulements plans 36, 37 qui viennent chacun en appui contre celui 19, 20 des rainures 17, 18 lors du serrage de la vis de pression 15 contre la tige de liaison 3.

En effet, le clip 14 comporte, dans sa partie supérieure et suivant une direction verticale, un alésage fileté 38 débouchant dans l'ouverture 30 et coopérant avec la vis de pression 15 pour permettre la fixation et l'immobilisation d'une part, du clip 14 dans le corps 7 du crochet 2 et d'autre part , de la tige de liaison 3 dans le crochet 2.

Le second crochet pédiculaire 4 du dispositif d'ancrage rachidien 1 comprend une partie d'ancrage osseux 40 se prolongeant par un corps ouvert 41 pour la réception et la fixation de la tige transversale 5.

La partie d'ancrage osseux 40 est constituée d'une lame 42 en forme de crochet s'étendant en dessous du corps 41 pour venir s'accrocher et se fixer sur une vertèbre 9 d'une colonne vertébrale et plus particulièrement sur un pédicule transverse 12 réséqué, se trouvant à l'opposé du premier 10 coopérant avec le crochet 2.

Le corps ouvert 41 du second crochet pédiculaire 4 comporte deux branches verticales et latérales 43, 44 délimitant une ouverture 45 en forme de U, comprenant une partie filetée 46 coopérant avec une vis de pression 47 pour le blocage dans le fond de l'ouverture 45 de la tige transversale 5.

Le dispositif d'ancrage rachidien 1 est fixé sur chaque vertèbre 9 du segment rachidien à instrumenter d'une colonne vertébrale après résection des pédicules transverses 10, 12 et de l'épineuse 39.

Le dispositif d'ancrage rachidien 1 est retenu sur chaque vertèbre 9 par une mise en compression des deux crochets pédiculaires 2, 4 en direction du centre de ladite vertèbre. Les crochets 2 et 4 viennent prendre appui respectivement sur la base des pédicules 10, 12 pour assurer une bonne tenue du dispositif d'ancrage rachidien 1.

La fixation et le blocage de la tige transversale 5 dans le logement allongé 25 du prolongement 24 du premier crochet pédiculaire 2 par la vis de pression 27 et dans l'ouverture 45 du second crochet pédiculaire 4 par la vis de pression 47 permet d'une part, de maintenir la compression des crochets pédiculaires 2, 4 en direction du centre de chaque vertèbre 9 et d'autre part, de retenir le dispositif d'ancrage rachidien 1.

Dès que les crochets 2 et 4 sont maintenus sur chaque vertèbre 9, il est prévu de disposer la tige de liaison 3 dans chaque ouverture 13 du corps 7 de chaque crochet 2 et de faire glisser chaque clip 14 sur la tige 3 afin de venir les encliqueter entre les branches 11 et 12 dudit corps 7. Le blocage en rotation et en translation de la tige de liaison 3 à l'intérieur de chaque crochet 2 est obtenu par le serrage de la vis de pression 15 qui permet également la retenue du clip 14 dans l'ouverture 13.

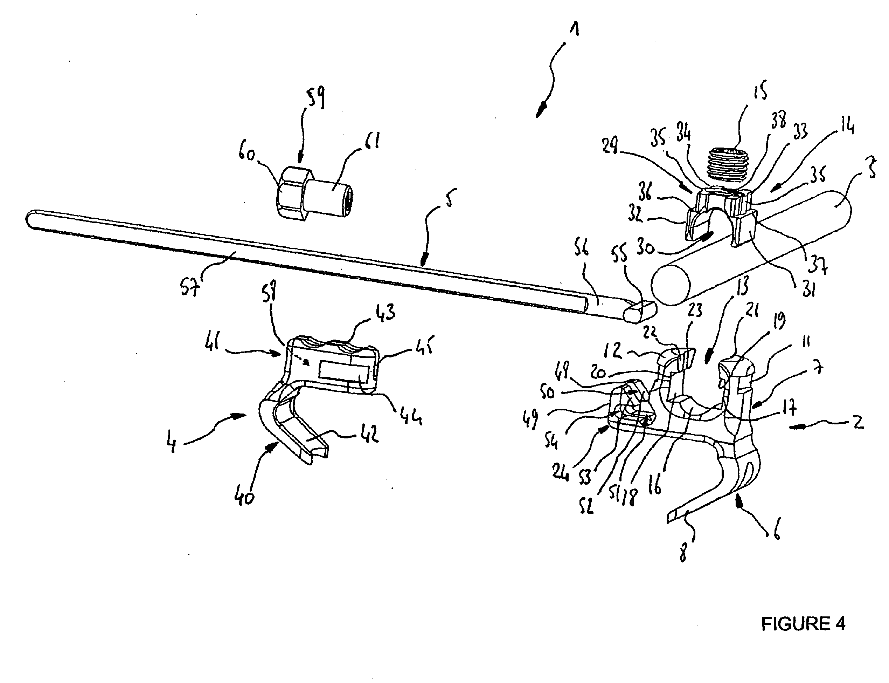

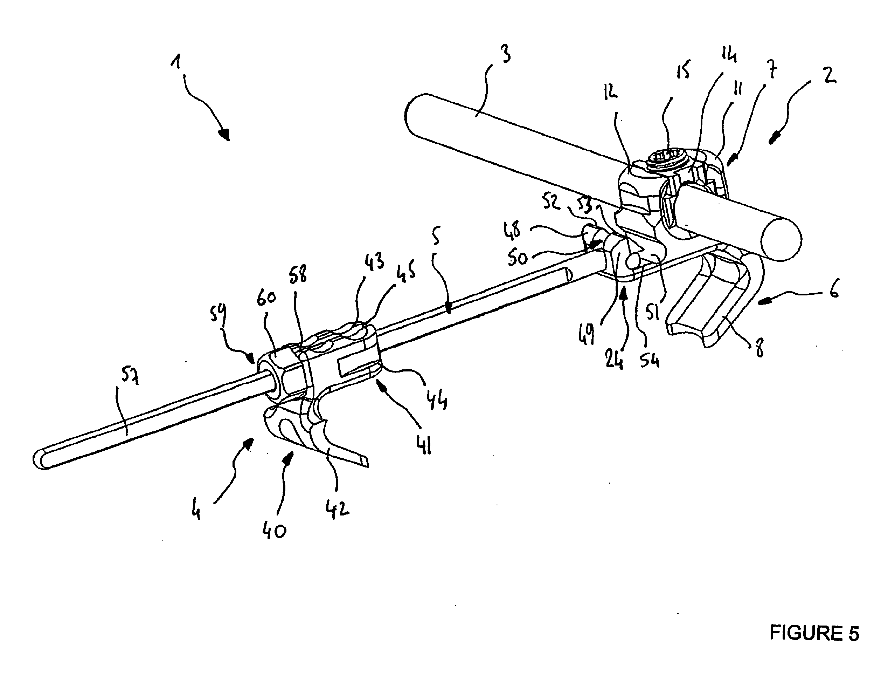

On a montré en figures 4 et 5 une première variante du dispositif d'ancrage rachidien 1 suivant la présente invention.

Cette variante se distingue de celle décrite précédemment en figures 1 à 3 principalement dans le profil du prolongement 24, de la tige transversale 5 et du second crochet pédiculaire 4, sachant que l'ensemble des autres éléments constituant le dispositif d'ancrage rachidien 1 est identique.

En effet, le premier crochet pédiculaire 2 comporte sur la face externe de sa branche verticale 12 du corps ouvert 7 un prolongement 24 dirigé d'une part perpendiculairement à la direction de fixation de la tige de liaison 3 dans l'ouverture 13 et d'autre part au dessus de la lame 8 formant crochet.

Le prolongement 24 présente un profil en forme de crochet constitué de deux lames parallèles 48, 49 séparées par une fente verticale 50 débouchant dans un logement 51. Chaque lame comporte un pan incliné 52, 53 formant la partie supérieure et externe des crochets et une partie d'accrochage 54 communiquant avec le logement 51.

La tige transversale 5 comprend une partie d'accrochage en forme de T constituée par un axe 55 disposé perpendiculairement à l'axe principal de la tige. La tige transversale 5 présente, à proximité de l'axe 55, des méplats parallèles 56 qui se prolongent par un profil cylindrique et fileté 57.

Le second crochet pédiculaire 4 comprend une partie d'ancrage osseux 40 se prolongeant par un corps ouvert 41 pour la réception et la fixation de la tige transversale 5.

Le corps ouvert 41 se distingue de celui décrit précédemment en ce que l'ouverture 45 en forme de U, délimitée par les branches verticales et latérales 43, 44 présente, à l'une des ses extrémités et suivant une direction horizontale, un alésage cylindrique 58 permettant la réception et le guidage axial d'un écrou de serrage 59.

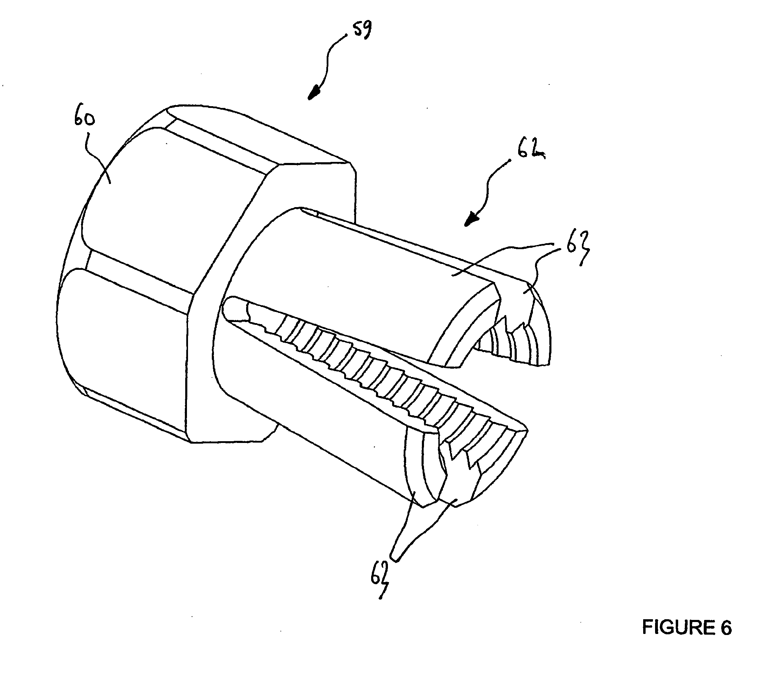

L'écrou 59 présente une tête de serrage 60 à profil hexagonal ou autre qui se prolonge par un cylindre creux 61 qui est fileté dans sa partie interne pour coopérer avec le profil fileté 57 de la tige transversale 5. En variante, l'écrou 59 peut présenter dans le prolongement de sa tête de serrage 60 un cylindre creux 62 fileté intérieurement et coupé longitudinalement en plusieurs portions 63 identiques (figure 6).

Cette variante permet de réaliser un écrou 59 à serrage rapide permettant de coulisser librement sur le profil cylindrique fileté 57 de la tige transversale 5 jusqu'au niveau du corps ouvert 41 du second crochet pédiculaire 4.

Ensuite, les portions 63 du cylindre creux 62 sont resserrées pour pouvoir pénétrer par vissage à l'intérieur de l'alésage 58 du corps ouvert 41 du second crochet pédiculaire 4.

Le montage du dispositif d'ancrage rachidien 1 décrit en figures 4 et 5 est semblable à celui décrit précédemment à savoir qu'il est fixé sur chaque vertèbre 9 d'un segment rachidien à instrumenter d'une colonne vertébrale après résection des pédicules transverses 10, 12 et de l'épineuse 39.

Le dispositif d'ancrage rachidien 1 est retenu sur chaque vertèbre 9 par une mise en compression des deux crochets pédiculaires 2, 4 en direction du centre de ladite vertèbre. Les crochets 2 et 4 viennent prendre appui respectivement sur la base des pédicules 10, 12 pour assurer une bonne tenue du dispositif d'ancrage rachidien 1.

Ainsi, la tige transversale 5 est introduite dans le logement 51 du prolongement 24 du premier crochet pédiculaire 2 de manière que l'axe 55 en forme de T coopère avec la partie d'accrochage 54 des lames 48, 49.

Lors du montage de la tige transversale 5 dans le prolongement 24 du premier crochet pédiculaire 2, on remarque que les méplats 56 coopèrent avec la fente verticale 50, prévue entre les lames 48, 49, afin de bloquer en rotation autour de son axe longitudinal la tige transversale 5.

La tige transversale 5 coopère, à l'opposé de son axe 55, avec l'ouverture 45 du corps ouvert 41 du second crochet pédiculaire 4. La mise en compression du dispositif d'ancrage rachidien 1 est réalisée par le serrage de l'écrou 59 sur la tige transversale 5 jusqu'à ce que son cylindre creux 61, 62 vienne à l'intérieur de l'alésage 58 du corps ouvert 41.

On note que l'écrou de serrage 59 permet d'une part, de faire varier la compression entre les crochets 2 et 4 et d'autre part, d'empêcher toute expulsion de la tige transversale 5 du second crochet 4 du fait de la coopération du cylindre creux 61, 62 avec l'alésage 58.

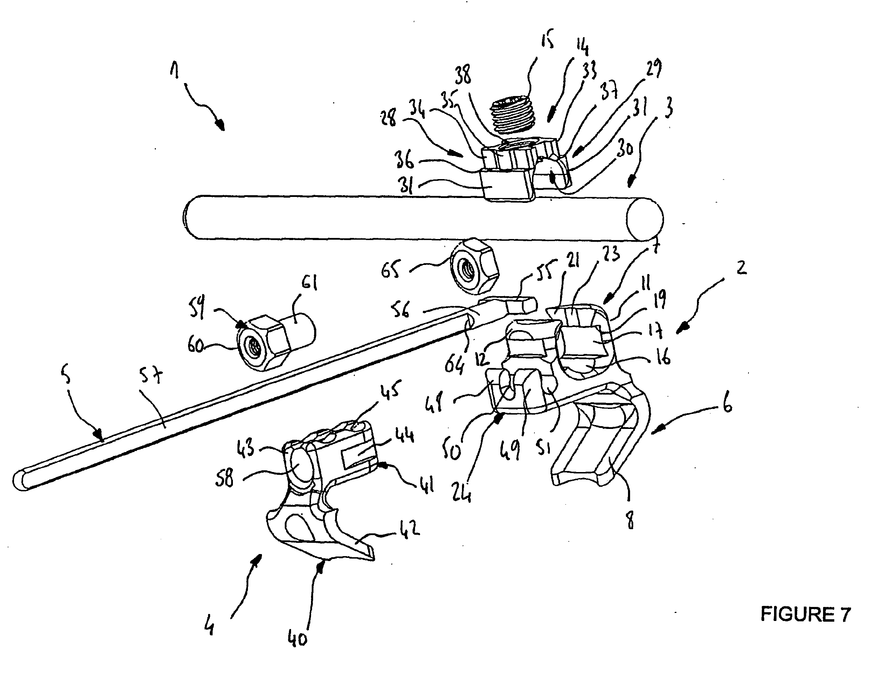

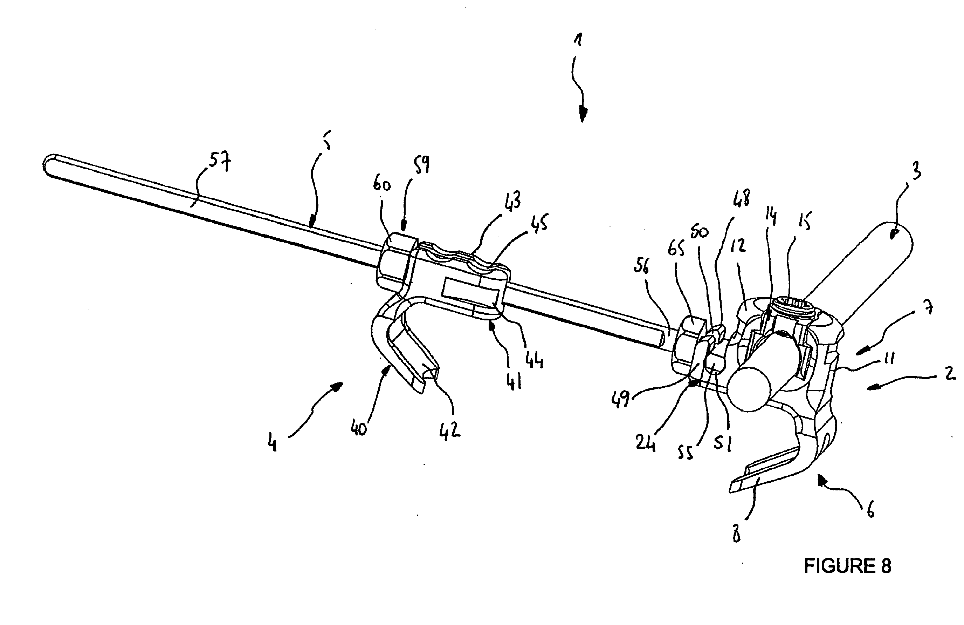

On a montré en figures 7 et 8 une seconde variante du dispositif d'ancrage rachidien 1 suivant la présente invention.

Cette variante se distingue de celle décrite précédemment en figures 4 à 6, principalement dans le profil du prolongement 24, et de la tige transversale 5 , sachant que l'ensemble des autres éléments constituant le dispositif d'ancrage rachidien 1 est identique.

En effet, le prolongement 24 se distingue de celui décrit et montré en figures 4 et 5 en ce que les lames verticales 48 et 49 ne comportent plus de pan inclinés 52, 53 et de partie d'accrochage 54. Par contre, les lames verticales 48 et 49 sont toujours séparées par une fente verticale 50 pour le passage de la tige transversale 5.

Egalement, le prolongement 24 comporte entre les lames 48 et 49 et la face externe de la branche verticale 12 du corps ouvert 7 un logement 51 dont les dimensions sont suffisantes pour recevoir l'axe 55 de la tige transversale 5 en forme de T.

D'autre part, la tige transversale 5 comporte au niveau de ses méplats 56 une partie filetée 64 coopérant avec un autre écrou de serrage 65 permettant de verrouiller le recul de la tige au niveau de son axe 55 en T par rapport au premier crochet pédiculaire 2.

Le montage du dispositif d'ancrage rachidien est semblable à celui décrit en figures 4 à 6 en ce qui concerne la mise en compression des crochets pédiculaires 2 et 4 sur une vertèbre 9 par l'intermédiaire de la tige transversale 5.

L'avantage de verrouiller le recul de la tige 5 au niveau de son axe 55 peut être appliqué à la variante décrite et montrée en figures 4 et 5.

Il doit d'ailleurs être entendu que la description qui précède n'a été donnée qu'à titre d'exemple et qu'elle ne limite nullement le domaine de l'invention dont on ne sortirait pas en remplaçant les détails d'exécutions décrits par tout autre équivalent.

## Revendications

1. Dispositif d'ancrage rachidien à crochets pédiculaires (2, 4), permettant la mise en place et la fixation d'une tige de liaison (3) au niveau de chaque vertèbre d'une colonne vertébrale; le dispositif comportant premier crochet pédiculaire (2) comprenant une partie d'ancrage osseux (6) et un corps ouvert (7) pour la réception et la fixation de la tige de liaison (3) par l'intermédiaire de moyen de fixation (14, 15), ledit corps ouvert (7) comportant sur sa face externe un prolongement (24) pourvu de moyens de fixation pour la réception et le blocage dans une direction sensiblement perpendiculaire à celle de la tige de liaison (3) d'une tige transversale (5), ledit prolongement (24) comportant deux lames parallèles (48, 49) séparées par une fente verticale (50) débouchant dans un logement (51) pour la réception de la tige transversale (5) et un second crochet pédiculaire (4) comprenant une partie d'ancrage osseux (40) et un corps ouvert (41) pourvu de moyens de fixation pour la réception et la fixation de la tige de la transversale (5) afin d'assurer une mise en compression des deux crochets pédiculaires (2, 4) en direction du centre de la vertèbre (9) correspondante.

2. Dispositif d'ancrage rachidien suivant la revendication 1, **caractérisé en ce que** chaque lame parallèle (48, 49) comporte un pan incliné (52, 53) formant la partie supérieure et externe d'un crochet et une partie d'accrochage (54) communiquant avec le logement (51) pour la réception de la tige transversale (5).

3. Dispositif d'ancrage rachidien suivant la revendication 1, **caractérisé en ce que** le prolongement (24) est dirigé d'une part perpendiculairement à la direction de fixation de la tige de liaison (3) et d'autre part au dessus de la partie d'ancrage osseux (6) du premier crochet pédiculaire (2),

4. Dispositif d'ancrage rachidien suivant la revendication 1, **caractérisé en ce que** le corps ouvert (41) du second crochet pédiculaire (4) comporte deux branches verticales et latérales (43, 44) délimitant une ouverture (45) en forme de U comprenant une partie filetée (46) coopérant avec une vis de pression (47) pour le blocage dans le fond de l'ouverture (45) de la tige transversale (5).

5. Dispositif d'ancrage rachidien suivant la revendication 1, **caractérisé en ce que** le corps ouvert (41) du second crochet pédiculaire (4) comporte deux branches verticales et latérales (43, 44) délimitant une ouverture (45) en forme de U comprenant, à l'une de ses extrémités et suivant une direction horizontale, un alésage cylindrique (58) permettant la réception et le guidage axial d'un écrou de serrage (59) pour le blocage de la tige transversale (5).

6. Dispositif d'ancrage rachidien suivant la revendication 1, **caractérisé en ce que** la tige transversale (5) présente un profil cylindrique sécable et lisse.

7. Dispositif d'ancrage rachidien suivant la revendication 1, **caractérisé en ce que** la tige transversale (5) comprend une partie d'accrochage en forme de T constituée par un axe (55), et à proximité de l'axe (55) des méplats parallèles (56) qui se prolongent par un profil cylindrique et fileté (57).

8. Dispositif d'ancrage rachidien suivant la revendication 7, **caractérisé en ce que** la tige transversale (5) comporte au niveau de ses méplats (56) une partie filetée (64) coopérant avec un écrou de serrage (65) permettant de verrouiller le recul de la tige au niveau de son axe (55) par rapport au premier crochet (2).

9. Dispositif d'ancrage rachidien suivant la revendication 5, **caractérisé en ce que** l'écrou (59) présente une tête de serrage (60) qui se prolonge par un cylindre creux (61) qui est fileté dans sa partie interne pour coopérer avec le profil fileté (57) de la tige transversale (5).

10. Dispositif d'ancrage rachidien suivant la revendication 9, **caractérisé en ce que** l'écrou (59) peut présenter dans le prolongement de sa tête de serrage (60) un cylindre creux (62) fileté intérieurement et coupé longitudinalement en plusieurs portions (63) identiques.

## Claims

1. Spinal fixing device with pedicle hooks (2, 4) for the placement and attachment of a connecting rod (3) at each vertebra (9) of a spinal column; the device comprising a first pedicle hook (2) comprising a bone anchor part (6) and an open body (7) to accept and attach the connecting rod (3) by means of an attaching means (14, 15), the said open body (7) comprising, on its external face, a continuation (24) provided with attachment means for accepting a transverse rod (5) and immobilizing same in a direction substantially perpendicular to that of the connecting rod (3), said continuation (24) comprising two parallel blades (48, 49) separated by a vertical slot (50) opening into a housing (51) to accept the transverse rod (5), and a second pedicle hook (4) comprising a bone anchor part (40) and an open body (41) provided with attachment means for accepting and attaching the transverse rod (5) so as to be able to compress the two pedicle hooks (2, 4) in the direction of the centre of the corresponding vertebra (9).

2. Spinal fixing device according to Claim 1, **characterized in that** each parallel blade (48, 49) comprises an inclined facet (52, 53) forming the upper and outer part of a hook and a fixing part (54) communicating with the housing (51) for accepting the transverse rod (5).

3. Spinal fixing device according to Claim 1, **characterized in that** the continuation (24) is directed, on the one hand, perpendicular to the direction of attachment of the connecting rod (3) and, on the other hand, above the bone anchor part (6) of the first pedicle hook (2).

4. Spinal fixing device according to Claim 1, **characterized in that** the open body (41) of the second pedicle hook (4) comprises two vertical and lateral branches (43, 44) delimiting a U-shaped opening (45) comprising a threaded part (46) engaging with a binding screw (47) for immobilizing the transverse rod (5) in the bottom of the opening (45).

5. Spinal fixing device according to Claim 1, **characterized in that** the open body (41) of the second pedicle hook (4) comprises two vertical and lateral branches (43, 44) delimiting a U-shaped opening (45) comprising, at one of its ends and in a horizontal direction, a cylindrical bore (58) for axially accepting and guiding a lock nut (59) for immobilizing the transverse rod (5).

6. Spinal fixing device according to Claim 1, **characterized in that** the transverse rod (5) has a sectile and plain cylindrical profile.

7. Spinal fixing device according to Claim 1, **characterized in that** the transverse rod (5) has a T-shaped fixing part consisting of a pin (55) and, near the pin (55), parallel flats (56) extended by a cylindrical and threaded profile (57).

8. Spinal fixing device according to Claim 7, **characterized in that** the transverse rod (5) has, in the region of its flats (56), a threaded part (64) that engages with a lock nut (65) which can be used to lock, in the region of the pin (55) thereof, the backward movement of the rod with respect to the first hook (2).

9. Spinal fixing device according to Claim 5, **characterized in that** the nut (59) has a head (60), used for tightening, extended by a hollow cylinder (61) which is threaded in its internal part to engage with the threaded profile (57) of the transverse rod (5).

10. Spinal fixing device according to Claim 9, **characterized in that** the nut (59) may, in the extension of its tightening head (60), have a hollow cylinder (62) which is internally threaded and split longitudinally into a number of identical portions (63).

## Patentansprüche

1. Vorrichtung zur Wirbelsäulenverankerung mit Pedikelhaken (2, 4), die die Anordnung und die Befestigung einer Verbindungsstange (3) im Bereich jedes Wirbels (9) einer Wirbelsäule ermöglicht; wobei die Vorrichtung einen ersten Pedikelhaken (2), der einen Knochenverankerungsbereich (6) und einen offenen Körper (7) zur Aufnahme und Befestigung der Verbindungsstange (3) über Befestigungseinrichtungen (14, 15) enthält, wobei der offene Körper (7) auf seiner Außenseite eine Verlängerung (24) aufweist, die mit Befestigungseinrichtungen zur Aufnahme und Blockierung einer Querstange (5) in einer Richtung im Wesentlichen lotrecht zu derjenigen der Verbindungsstange (3) versehen ist, wobei die Verlängerung (24) zwei parallele Lamellen (48, 49) aufweist, die durch einen senkrechten Schlitz (50) getrennt sind, der in einen Sitz (51) zur Aufnahme der Querstange (5) mündet, und einen zweiten Pedikelhaken (4) aufweist, der einen Knochenverankerungsbereich (40) und einen offenen Körper (41) enthält, der mit Befestigungseinrichtungen zur Aufnahme und Befestigung der Querstange (5) versehen ist, um eine Komprimierung der zwei Pedikelhaken (2, 4) in Richtung der Mitte des entsprechenden Wirbels (9) zu gewährleisten.

2. Vorrichtung zur Wirbelsäulenverankerung nach Anspruch 1, **dadurch gekennzeichnet, dass** jede parallele Lamelle (48, 49) eine geneigte Fläche (52, 53), die den oberen und äußeren Bereich eines Hakens bildet, und einen Befestigungsbereich (54) aufweist, der mit dem Sitz (51) zur Aufnahme der Querstange (5) in Verbindung steht.

3. Vorrichtung zur Wirbelsäulenverankerung nach Anspruch 1, **dadurch gekennzeichnet, dass** die Verlängerung (24) einerseits lotrecht zur Befestigungsrichtung der Verbindungsstange (3) und andererseits oberhalb des Knochenverankerungsbereichs (6) des ersten Pedikelhakens (2) gerichtet ist.

4. Vorrichtung zur Wirbelsäulenverankerung nach Anspruch 1, **dadurch gekennzeichnet, dass** der offene Körper (41) des zweiten Pedikelhakens (4) zwei senkrechte und seitliche Zweige (43, 44) aufweist, die eine U-förmige Öffnung (45) begrenzen, welche einen Gewindebereich (46) enthält, der mit einer Druckschraube (47) zum Blockieren der Querstange (5) am Boden der Öffnung (45) zusammenwirkt.

5. Vorrichtung zur Wirbelsäulenverankerung nach Anspruch 1, **dadurch gekennzeichnet, dass** der offene Körper (41) des zweiten Pedikelhakens (4) zwei senkrechte und seitliche Zweige (43, 44) aufweist, die eine U-förmige Öffnung (45) begrenzen, welche an einem ihrer Enden und in einer waagrechten Richtung eine zylindrische Bohrung (58) enthält, die die Aufnahme und die axiale Führung einer Spannmutter (59) zur Blockierung der Querstange (5) erlaubt.

6. Vorrichtung zur Wirbelsäulenverankerung nach Anspruch 1, **dadurch gekennzeichnet, dass** die Querstange (5) ein teilbares und glattes zylindrisches Profil aufweist.

7. Vorrichtung zur Wirbelsäulenverankerung nach Anspruch 1, **dadurch gekennzeichnet, dass** die Querstange (5) einen aus einer Achse (55) bestehenden T-förmigen Befestigungsbereich und in der Nähe der Achse (55) parallele Abflachungen (56) enthält, die sich in einem zylindrischen und mit Gewinde versehenen Profil (57) fortsetzen.

8. Vorrichtung zur Wirbelsäulenverankerung nach Anspruch 7, **dadurch gekennzeichnet, dass** die Querstange (5) im Bereich ihrer Abflachungen (56) einen Gewindebereich (64) enthält, der mit einer Spannmutter (65) zusammenwirkt, die es ermöglicht, den Rückstoß der Stange im Bereich ihrer Achse (55) bezüglich des ersten Hakens (2) zu verriegeln.

9. Vorrichtung zur Wirbelsäulenverankerung nach Anspruch 5, **dadurch gekennzeichnet, dass** die Mutter (59) einen Spannkopf (60) aufweist, der sich in einem Hohlzylinder (61) verlängert, welcher innen mit Gewinde versehen ist, um mit dem Gewindeprofil (57) der Querstange (5) zusammenzuwirken.

10. Vorrichtung zur Wirbelsäulenverankerung nach Anspruch 9, **dadurch gekennzeichnet, dass** die Mutter (59) in der Verlängerung ihres Spannkopfes (60) einen Hohlzylinder (62) aufweisen kann, der mit einem Innengewinde versehen und in Längsrichtung in mehrere gleiche Abschnitte (63) geschnitten ist.
